(19) Europäisches Patentamt
European Patent Office
Office européen des brevets

(11) **EP 3 375 375 A1**

(12) **EUROPEAN PATENT APPLICATION**

(43) Date of publication:
**19.09.2018 Bulletin 2018/38**

(51) Int Cl.:
***A61B 6/03*** (2006.01)          ***A61B 6/00*** (2006.01)
***G21K 1/10*** (2006.01)

(21) Application number: **17161362.3**

(22) Date of filing: **16.03.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **PROKSA, Roland**
  **5656 AE Eindhoven (NL)**
• **GLEICH, Bernhard**
  **5656 AE Eindhoven (NL)**
• **NOEL, Peter Benjamin**
  **5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **X-RAY DEVICE**

(57)     The invention relates to an x-ray device for providing x-rays. The x-ray device 20 comprises an x-ray source 2 for generating x-rays and a grating device for absorbing at least a part of the x-rays, wherein the grating device 3 has lamellae comprising an x-ray absorbing material. At least one of the x-ray source 2 and the grating device 3 is adapted for modifying an angular orientation of the x-rays relative to the lamellae. By modifying the angular orientation of the x-rays relative to the lamellae, the ways, along which the x-rays traverse the lamellae, are modified, i.e. the absorption of the x-rays by the lamellae is modified. This modified absorption leads to a modified energy distribution of the x-rays such that by modifying the angular orientation of the x-rays relative to the lamellae different energy spectra of the x-rays can be realized in a technically relatively simple way.

FIG. 1

**Description**

FIELD OF THE INVENTION

The invention relates to an x-ray device and method for providing x-rays. The invention relates further to an imaging system for generating an image of an object, which comprises the x-ray device, and a corresponding imaging method for generating an image of an object. Moreover, the invention relates to computer programs for controlling the x-ray device and the imaging system.

BACKGROUND OF THE INVENTION

An imaging system for generating an image of an object is, for instance, a computed tomography system having two x-ray tubes operated at different voltages, in order to provide x-rays having different energy distributions which traverse the object. The computed tomography system further comprises two detectors for detecting the x-rays after having traversed the object. In particular, one of the x-ray tubes is arranged opposite to one of the detectors on a rotating gantry and the other of the x-ray tubes is arranged opposite to the other of the detectors on the rotating gantry. While the gantry rotates around the object, the x-ray tubes generate the x-rays which traverse the object and the detectors detect the x-rays after having traversed the object and generate detection values depending on the detected x-rays, wherein a reconstruction unit reconstructs a computed tomography image based on the generated detection values.

Using two x-ray tubes and two detectors for generating detection values, which correspond to different energy distributions of the x-rays, leads to relatively high technical efforts.

SUMMARY OF THE INVENTION

It is an object of the present invention to provide an x-ray device which allows for a provision of x-rays having different energy distributions with less technical efforts. Moreover, it is an object of the present invention to provide a corresponding method for providing the x-rays, a computer program for controlling the x-ray device, an imaging system for generating an image of an object, which comprises the x-ray device, a corresponding imaging method for generating an image of an object, and a computer program for controlling the imaging system.

In a first aspect of the present invention an x-ray device for providing x-rays is presented, wherein the x-ray device comprises:

- an x-ray source for generating x-rays, and
- a grating device for absorbing at least a part of the x-rays, wherein the grating device has lamellae comprising an x-ray absorbing material,

wherein at least one of the x-ray source and the grating device is adapted for modifying an angular orientation of the x-rays relative to the lamellae.

By modifying the angular orientation of the x-rays relative to the lamellae, the ways, along which the x-rays traverse the lamellae, are modified, i.e. the absorption of the x-rays by the lamellae is modified. This modified absorption leads to a modified energy distribution of the x-rays such that by modifying the angular orientation of the x-rays relative to the lamellae different energy spectra of the x-rays can be realized in a technically relatively simple way.

It should be noted that the expression "at least one of A and B" includes i) A without B, ii) B without A, and iii) A and B. For instance, the expression "at least one of the x-ray source and the grating device is adapted for modifying an angular orientation of the x-rays relative to the lamellae" includes that i) the x-ray source is adapted for modifying an angular orientation of the x-rays relative to the lamellae, but not the grating device, ii) the grating device is adapted for modifying an angular orientation of the x-rays relative to the lamellae, but not the x-ray source, and iii) the x-ray source and the grating device are adapted for modifying an angular orientation of the x-rays relative to the lamellae.

The x-ray source is preferentially an x-ray tube. Preferentially, the x-ray source is adapted to generate divergent x-rays and the lamellae diverge. Moreover, at least one of the x-ray source and the grating device may be adapted for modifying the angular orientation of the x-rays relative to the lamellae from a first angular orientation, in which the x-rays are parallel to the lamellae, to a second angular orientation, in which the x-rays are not parallel to the lamellae, and vice versa.

In an embodiment at least one of the x-ray source and the grating device is adapted to modify the angular orientation such that a variation of penetration lengths through the lamellae of all generated x-rays is minimized. Thus, at the modified angular orientation differences between the penetration lengths of different rays through the lamellae are minimized. In particular, the variance of the penetration lengths is minimized. This minimization can be such that all generated x-rays have substantially the same penetration length through the lamellae. The spectrum of the x-rays can therefore be very homogeneous in space. The respective angular orientation can correspond to a local minimum or to a global minimum, i.e. with changing angular orientation the penetration lengths and hence the differences between the penetration lengths and variance change, wherein the angular orientations of the group can correspond to local or global minima of, for instance, the variance.

In an embodiment at least one of the x-ray source and the grating device is adapted for setting an angular orientation of the x-rays relative to the lamellae, which is selected from a group of predefined angular orientations, wherein for each angular orientation of this group the variation of penetration lengths through the lamellae of all generated x-rays has a local minimum, in particular for each angular orientation the x-rays have substantially a same penetration length through the lamellae. Thus, it can be switched between different energy distributions of the x-rays, wherein each energy distribution can be very homogeneous in space. In addition to this group of predefined angular orientations a further angular orientation can be provided, in which the x-rays are parallel to the lamellae. Preferentially, the lamellae are configured such that the respective x-ray, which passes through the respective entire length of the respective lamella in the parallel case, is completely absorbed such that it does not contribute to the x-ray spectrum which has passed the grating device. This means that in the parallel situation all x-rays, which contribute to the x-ray spectrum behind the grating device, have not been absorbed by the lamellae and hence all have the same penetration length zero, i.e. also in the parallel case the spectrum can be very homogeneous in space. The group of predefined angular orientations, wherein for each angular orientation of this group the variation of penetration lengths through the lamellae of all generated x-rays has a local minimum, can also have a single member only, wherein this single member can be provided together with the parallel case such that it can be switched between this single member and the parallel case, in order to provide two different angular orientations, wherein preferentially also in the parallel case the spectrum is very homogeneous in space.

In an embodiment at least one of the x-ray source and the grating device is adapted for setting an angular orientation of the x-rays relative to the lamellae, which is selected from a group of predefined angular orientations, wherein the angular orientation of a respective group is defined by

$$\alpha = \tan(np/h) \qquad ,$$

wherein $\alpha$ is the angle enclosed by a) an x-ray of the generated x-rays and b) the lamellae, $n$ is a natural number, $p$ is the pitch of the grating and $h$ is the height of the lamellae. The term "natural number" refers to all non-negative numbers including zero. Thus, $n$ can be, for instance, 0 and 1, in order to define two angular orientations. In an embodiment following $n$ values are used: $n = 1,2,3,...,N$, wherein $N$ indicates the number of angular orientations of the group. In another embodiment following $n$ values are used: $n = 0,1,2,3,..., N$, wherein $N + 1$ indicates the number of angular orientations of the group. The members of the group can also be defined by non-consecutive natural numbers $n$. By using these angular orientations for each angular orientation the energy distribution can be very homogeneous in space. The angle $\alpha$ can be defined between a center x-ray within the generated x-rays and the lamellae or between another x-ray of the generated x-rays and the lamellae.

Preferentially the x-ray source comprises a focal spot on a metal surface, from which the x-rays emanate, wherein the x-ray source is adapted to move the focal spot for modifying the angular orientation of the x-rays relative to the lamellae. The focal spot can be moved without necessarily requiring any mechanical movement of components of the x-ray device. The movement can therefore be carried out very fast such that a very fast switching between different energy distributions of the x-rays can be provided.

Preferentially the x-ray source comprises an electron beam generation unit for generating an electron beam directed on a region on the metal surface, thereby generating the focal spot from which the x-rays emanate, wherein in an embodiment the x-ray source is adapted to move the focal spot by moving the electron beam. In particular, the x-ray source can be adapted to move the electron beam by at least one of electrostatic and magnetic electron beam deflection. This allows moving the focal spot very fast with relatively low technical efforts.

In an embodiment the x-ray source comprises an electron beam generation unit for generating several electron beams and a switch for switching between the several electron beams, wherein the different electron beams are directed on different regions on a metal surface for generating different focal spots, from which the x-rays are emanatable, at different locations, wherein the x-ray source is adapted to provide a movable focal spot by switching between the different electron beams. This can lead to a further increased speed of switching between different energy distributions of the x-rays. The switch is preferentially a grid switch, i.e. it comprises a grid being charged such that a respective electron beam reaching the metal surface is not generated.

In an embodiment the x-ray source and the lamellae are mechanically moveable relative to each other, in order to modify the angular orientation of the x-rays relative to the lamellae. For instance, the grating device can be adapted to tilt the lamellae for modifying the angular orientation of the x-rays relative to the lamellae. By mechanically moving the x-ray source and the lamellae relative to each other relatively large changes of the angular orientation can be achieved in a technically relatively simple way.

In an embodiment the lamellae are moveable from a traversing position, in which the x-rays traverse the lamellae, to a non-traversing position, in which the x-rays do not traverse the lamellae, and vice versa. Thus, the lamellae can be moved out of the x-rays and into the x-rays, in order to use the lamellae only if desired. For instance, a sequence of different acquisitions of detection values can be carried out, wherein in a certain acquisition period of the sequence the lamellae may be used and wherein in another acquisition period of the sequence the lamellae may not be used.

In an embodiment the grating device comprises several sets of lamellae, wherein the lamellae of each set diverge such that fictive extensions of the lamellae meet at a respective fictive lamellae focus point, wherein different sets of lamellae have the same lamellae focus point or different lamellae focus points. A respective set of lamellae can be regarded as being a respective grating, wherein the x-ray device can be adapted to select one or several of the several gratings for generating the detection values. For instance, in an acquisition period one of these gratings and in another acquisition period another of these gratings might be used. The different gratings have preferentially different properties, wherein these properties may be selected from a group consisting of lamella thickness, lamella height, lamellae period and lamella material. In an embodiment a first grating with tin lamellae and a second grating with gold lamellae is used. Tin has a relatively low K-edge energy such that the output spectrum contains mainly high energy photons. Gold has a relatively high K-edge energy and provides mainly low energy photons at the output. Such a combination can especially be useful, if the x-ray device is used in an imaging system for imaging a contrast agent within a subject, wherein the contrast agent has a K-edge energy in between the K-edge energies of tin and gold like iodine.

Also if the lamellae of the different sets of lamellae do not diverge or do not diverge such that fictive extensions of the lamella meet at a respective lamella focus point, the x-ray device can comprise several sets of lamellae having different properties. By providing several sets of lamellae, which can be selectively used for acquiring detection values being indicative of the x-rays after having traversed an object to be imaged, the energy distribution of the x-rays cannot only be modified by modifying the angular orientation of the x-rays relative to the lamellae, but also by changing the set of lamellae traversed by the x-rays. This can lead to a larger providable range of different energy spectra of the x-rays.

In an embodiment the lamellae focus points are on the metal surface, wherein the x-ray source is adapted to move the focal spot from a position, at which it meets the lamellae focus point of one of the sets of lamellae, to another position, at which it meets the lamellae focus point of another of the sets of lamellae, and vice versa. This allows switching between different sets of lamellae, i.e. between different gratings, or between predominant uses of different sets of lamellae by moving the focal spot to a respective lamellae focus point. This can allow for a switching between different gratings or between different predominant uses without necessarily requiring a mechanical movement such that this switching can be carried out very fast. The focal spot may be moved by moving an electron beam used for generating the x-rays or by switching between different electron beams. Also in this embodiment a first grating with tin lamellae and a second grating with gold lamellae might be used.

In an embodiment the x-ray source comprises an electron beam generation unit for generating an electron beam between a cathode and an anode, which is directed on a region on a metal surface of the anode, thereby generating the focal spot from which the x-rays emanate, wherein the x-ray source is adapted to change at least one of a voltage and a current between the cathode and the anode. Thus, the energy spectrum of the x-rays can also be modified by modifying the voltage and/or the current between the cathode and the anode. For instance, while acquiring detection values being indicative of the x-rays, the voltage and/or the current can be modified, in order to provide x-rays having different energy distributions. This can further increase the range of providable energy distributions of the x-rays.

The x-ray device can be adapted to not provide x-rays while modifying the energy distribution, in order to only provide x-rays after this modification process has been completed. This allows for providing an accurate known energy distribution which can lead, for instance, to an improved reconstruction of a computed tomography image based on the detection values, if a reconstruction algorithm used for the reconstruction assumes that a certain energy spectrum was present when acquiring the detection values.

In a further aspect of the present invention an imaging system for generating an image of an object is presented, wherein the imaging system comprises:

- an x-ray device as defined in claim 1 for providing x-rays for traversing the object,
- an x-ray detector for generating detection data depending on the x-rays after having traversed the object, and
- an image generating unit for generating the image based on the detected x-rays.

In another aspect of the present invention a method for providing x-rays by using the x-ray device as defined in claim 1 is presented, wherein the method comprises generating x-rays by the x-ray source, wherein an angular orientation of the x-rays relative to the lamellae is modified.

In a further aspect of the present invention an imaging method for generating an image of an object is presented, wherein the imaging method comprises:

- providing x-rays for traversing the object by an x-ray device as defined in claim 1,
- generating detection data depending on the x-rays after having traversed the object by an x-ray detector, and
- generating the image based on the detected x-rays by an image generating unit.

In another aspect of the present invention a computer program for controlling an x-ray device as defined in claim 1 is presented, wherein the computer program comprises program code means for causing the x-ray device to carry out the method as defined in claim 12, when the computer program is run on a controller controlling the x-ray device.

In a further aspect of the present invention a computer program for controlling an imaging system as defined in claim 1 is presented, wherein the computer program comprises program code means for causing the imaging system to carry out the imaging method as defined in claim 13, when the computer program is run on a controller controlling the imaging system.

It shall be understood that the x-ray device of claim 1, the imaging system for generating an image of claim 11, the method for providing x-rays of claim 12, the imaging method for generating an image of claim 13, computer program for controlling an x-ray device of claim 14 and the computer program for controlling an imaging system of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily an embodiment of an imaging system for generating an image of an object,
Fig. 2 illustrates schematically and exemplarily a first angular orientation of x-rays relative to lamellae of a grating,
Fig. 3 illustrates schematically and exemplarily a further angular orientation of the x-rays relative to the lamellae of the grating,
Fig. 4 illustrated schematically and exemplarily an embodiment of an x-ray source,
Fig. 5 illustrates schematically and exemplarily a movement of a focal spot on an anode of the x-ray source along a rotational direction with respect to a rotation of the anode,
Fig. 6 illustrates schematically and exemplarily a radial movement of the focal spot on the anode of the x-ray source,
Fig. 7 illustrates schematically and exemplarily a further embodiment of an x-ray source,
Fig. 8 illustrates schematically and exemplarily a movement of a grating relative to x-rays,
Fig. 9 illustrates schematically and exemplarily an x-ray device having several gratings to be traversed by x-rays,
Fig. 10 illustrates schematically and exemplarily a grating with divergent lamellae,
Fig. 11 illustrates schematically and exemplarily a selection of a predominantly used grating based on a focal spot movement, and
Fig. 12 shows a flowchart exemplarily illustrating an embodiment of an imaging method for generating an image of an object.

DETAILED DESCRIPTION OF EMBODIMENTS

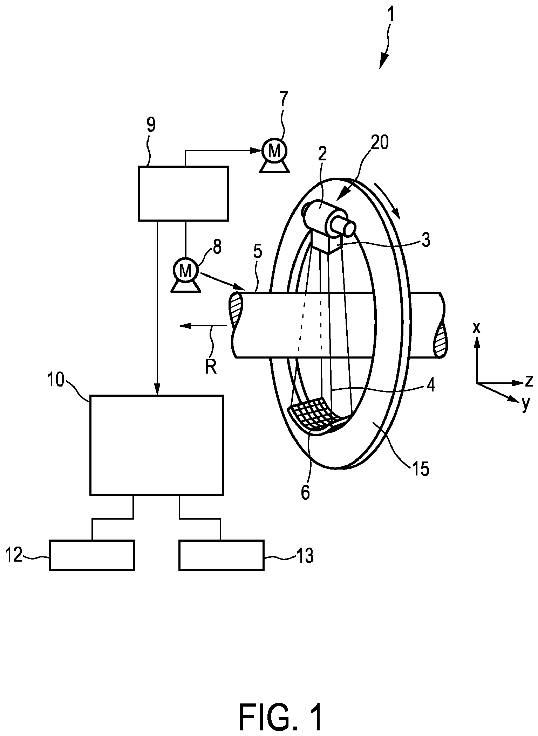

Fig. 1 shows schematically and exemplarily an embodiment of an imaging system for generating an image of an object. In this embodiment the imaging system 1 is a spectral computed tomography system for generating a computed tomography image of a person. The computed tomography system 1 includes a gantry 15 which is capable of rotating around a rotational axis R which extends parallel to a z direction. A radiation device 20 for providing x-rays 4 is mounted on the gantry 15. The x-rays traverse the person (not shown in Fig. 1) within an imaging region 5, which can also be regarded as being an examination zone and which, in this embodiment, is cylindrical. After having traversed the imaging region 5, the x-rays 4 are incident on a detector 6 comprising a two-dimensional detection surface, wherein the detector 6 is also mounted on the gantry 15.

The spectral computed tomography system 1 comprises two motors 7, 8. The gantry 1 is driven at a preferably constant but adjustable angular speed by the motor 7. The motor 8 is provided for displacing the person, who is arranged on a support means like a table within the imaging region 5, parallel to the direction of the rotational axis R or the z axis. These motors 7, 8 are controlled by a control unit 9, for instance, such that the x-ray device 20 and the person move relatively to each other along a helical trajectory. However, it is also possible that the x-ray device 20 and the person move relatively to each other along another trajectory. For instance, the person might not be moved and only the x-ray device 20 may be rotated such that the x-ray device 20 moves along a circular trajectory relative to the person. Furthermore, although the x-rays 4 schematically and exemplarily shown in Fig. 1 form a cone beam, in another embodiment they can form another beam having another shape like a fan beam, wherein the detector 6 can comprise a detection surface being adapted corresponding to the other beam shape, in particular to the fan beam.

During a relative movement of the x-ray device 20 and the person, the detector 6 generates detection values being indicative of the x-rays after having traversed the person, wherein detection values generated at a same time are regarded as forming a projection.

The x-ray device 20 comprises an x-ray source 2 like an x-ray tube for generating x-rays and a grating device 3 for absorbing at least a part of the x-rays, wherein the grating device 3 has lamellae comprising an x-ray absorbing material and wherein at least one of the x-ray source 2 and the grating device 3 is adapted for modifying an angular orientation of the x-rays relative to the lamellae, in order to provide x-rays having different energy spectra. In particular, by modifying the angular orientation of the x-rays relative to the lamellae, different sets of projections can be acquired, which correspond to different energy distributions, i.e. spectral projections can be acquired.

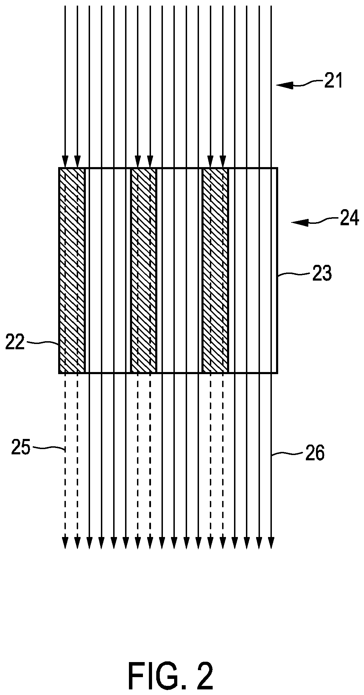

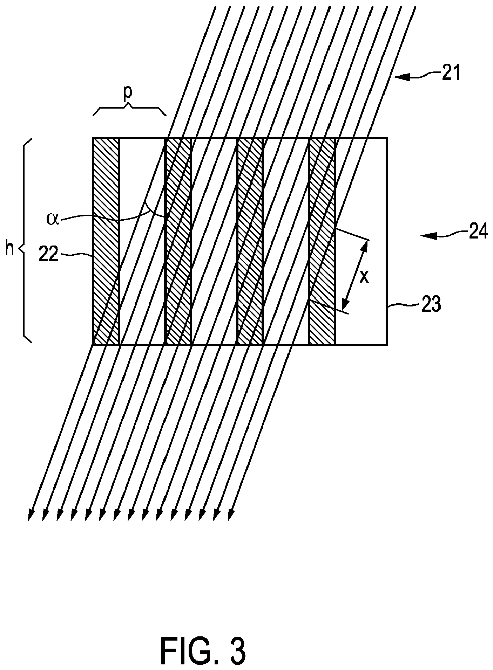

Figs. 2 and 3 schematically and exemplarily illustrate how the absorption and hence the energy spectrum changes depending on an angular orientation of x-rays 21 relative to lamellae 22. As can be seen in these figures, the lamellae 22 are separated by spacers 23, wherein the lamellae 22 together with the spacers 23 form a grating 24. The grating 24 is preferentially a fine pitch absorber grating with a high aspect ratio. The lamellae 22 can have a thickness of, for instance, 10 μm and a height of, for instance, 2 mm, wherein the spacers 23 can form a matrix which does substantially not absorb the x-rays 21. The grating period might be, for example, 50 μm. If the x-rays 21 pass the grating 24 parallel to the grating lamellae as schematically and exemplarily illustrated in Fig. 2, in this example 80 percent of the x-rays undergo no or only little absorption in the spacers 23 and these x-rays 26 dominate the output spectrum. The other 20 percent of the x-rays are strongly attenuated by the lamellae 22 resulting in x-rays 25 having only a very little or no impact on the output spectrum. Thus, the absorbed x-rays 25 do substantially not contribute to the spectrum behind the grating 24, whereas the x-rays 26 substantially contribute to the spectrum behind the grating 24 and have a same penetration length of zero through the lamellae 22 such that the spectrum can be very homogeneous in space. This attenuation behavior changes significantly if the x-rays 21 are angulated, even slightly angulated, relative to the lamellae 22 as schematically and exemplarily illustrated in Fig. 3.

In Fig. 3 the x-rays 21 are angularly rotated with respect to the lamellae 22 such that each x-ray has a same penetration length x through the lamellae 22 which can also be regarded as being filter lamellae. Thus, in Fig. 3 a variation of penetration lengths through the lamellae 22 of all generated x-rays is minimized. The same penetration length x also causes a homogeneous but very different output spectrum compared with the non-angulated case shown in Fig. 2.

Preferentially, at least two different angular orientations of the x-rays relative to the lamellae are provided, wherein during the acquisition of the spectral projections it is switched between these different angular orientations. In these different angular orientations the penetration length for each x-ray, which contributes to the filtered spectrum, is preferentially substantially the same such that the energy distribution is relatively homogeneous in space. In particular, at least one of the x-ray source 2 and the grating device 3 is adapted for setting an angular orientation of the x-rays 21 relative to the lamellae 22, which is selected from a group of predefined angular orientations, wherein for each angular orientation of this group the variation of penetration lengths through the lamellae 22 of all generated x-rays 21 has a local minimum, in particular for each angular orientation the x-rays 21 have substantially a same penetration length through the lamellae 22. Thus, it can be switched between different energy distributions of the x-rays 21, wherein each energy distribution can be very homogeneous in space.

Moreover, in an embodiment at least one of the x-ray source 2 and the grating device 3 is adapted for setting an angular orientation of the x-rays 21 relative to the lamellae 22, which is selected from a group of predefined angular orientations, wherein the angular orientation of a respective group is defined by

$$\alpha = \tan(np/h) \qquad ,$$

wherein $\alpha$ is the angle enclosed by a) an x-ray of the generated x-rays and b) the lamellae, $n = 0,1,2,3,..., N$ with $N + 1$ being the number of angular orientations of the group, $p$ is the pitch of the grating and $h$ is the height of the lamellae. By using these predefined angular orientations, for each angular orientation the energy distribution can be very homogeneous in space.

It should be noted that in Figs. 2 and 3 the x-rays 21 and the lamellae 22 are parallel only for demonstration purposes, i.e. in order to explain that the attenuation behavior and hence the energy spectrum changes with changing angular orientation. Although a spectral computed tomography system can comprise parallel x-rays and parallel lamellae, in the embodiment described with reference to Fig. 1 the x-rays diverge and also the lamellae diverge. Implementations with divergent x-rays from a finite size focal spot and a range of angular orientations between the divergent x-rays and divergent lamellae show the same effect, i.e. also show the effect illustrated above with references to Figs. 2 and 3. In case of divergent rays the angle $\alpha$ can be defined between a center x-ray within the generated x-rays and the lamellae or between another x-ray of the generated x-rays and the lamellae. The significant change of the spectral filtration caused by the change of the attenuation behavior can be used for fast and simple switching of the spectral filtration. Several technical ways to realize the required angulation of the grating relative to the x-rays will be explained in the following.

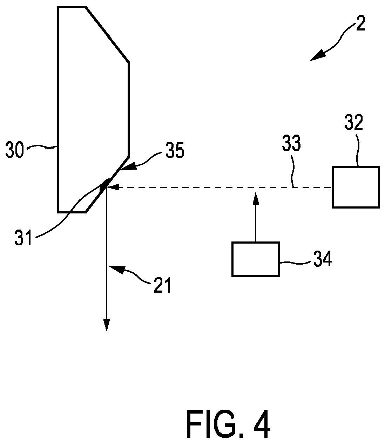

Fig. 4 schematically and exemplarily illustrates an embodiment of the x-ray source 2 having an anode 30 and a cathode 32 for generating an electron beam 33 in a known way. The anode 30 is rotating and the electron beam 33 is directed on a region on a metal surface 35, in order to generate a focal spot 31 from which the x-rays 21 emanate. The x-ray source 2 comprises an electron beam deflection unit 34 for deflecting the electron beam 33, in order to move the electron beam 33 and hence the focal spot 31. The electron beam deflection unit 34 is preferentially adapted to electrostatically or magnetically deflect the electron beam 33.

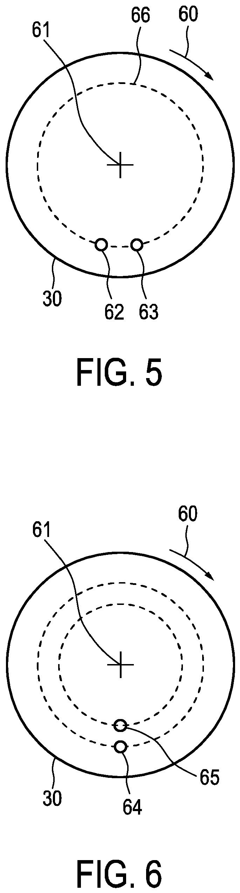

By moving the focal spot 31 the position from which the x-rays 21 emanate is modified, wherein this modification leads to a change of the angular orientation of the x-rays 21 relative to the lamellae 22. Thus, by controlling the electron beam deflection unit 34 the angular orientation of the x-rays 21 relative to the lamellae 22 can be controlled. For controlling the x-ray source 2, particularly the electron beam deflection unit 34, the control unit 9 or another control unit can be used. The electron beam deflection unit 34 can be adapted to deflect the electron beam 33 to provide a focal spot movement in different directions. For instance, with respect to the rotating anode 30 the focal spot 31 may be moved radially, along a rotation direction of the rotating anode 30, or radially and along the rotation direction, i.e. diagonally. In the following the terms "radially" and "along the rotation direction" will be described with reference to Figs. 5 and 6.

The anode 30 rotates around a rotational axis 61 in the rotation direction 60. Fig. 5 schematically and exemplarily illustrates a movement of the focal spot from a first position 63 to a second position 62 on the rotating anode 30 along the rotation direction 60. As can be seen in Fig. 5, this movement of the focal spot can be regarded as being a movement along a circular line 66 which runs through the focal spot to be moved and which is centered on the mid of the rotating anode 30 which corresponds to the position of the rotational axis 61. Fig. 6 schematically and exemplarily illustrates a radial movement of a focal spot from a first position 64 to a second position 65.

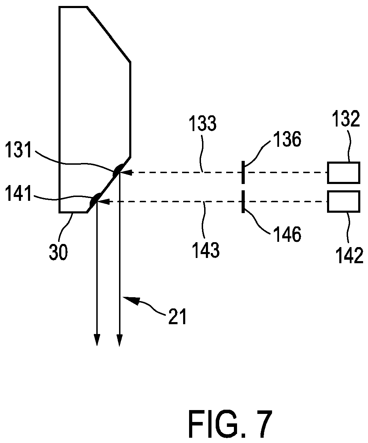

A further embodiment of the x-ray source 2 is schematically and exemplarily illustrated in Fig. 7. In this embodiment the x-ray source comprises two cathodes 132, 142 and a rotating anode 30 for generating two electron beams 133, 143. The x-ray source further comprises grid switches 136, 146 for switching the electron beams 133, 143 on and off. If the respective electron beam 133, 143 is switched on, it generates a respective focal spot 131, 141 on the metal surface of the anode 30 for generating the x-rays 21. If a first electron beam 133 of the two electron beams is switched on, the focal spot 131 is at a first position and, if a second electron beam 143 of the electron beams is switched on, the x-rays 21 emanate from the focal spot 141 at a second position. Thus, by switching between the electron beams 133, 143 the focal spot, from which the x-rays emanate, can be moved between the first and second positions on the metal surface of the anode 30. Also this grid-switch-based movement of the focal spot leads to a change of the angular orientation of the x-rays relative to the lamellae and hence to a change in the energy spectrum of the x-rays. Also the grid switches 136, 146 can be controlled by the control unit 9 or by another control unit, in order to control the different energy spectra of the x-rays.

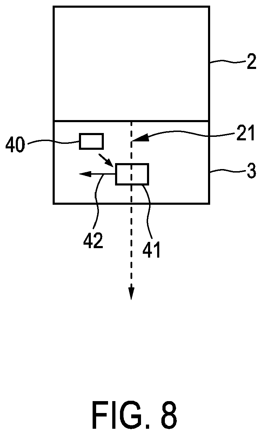

In an embodiment the x-ray source and the lamellae can be mechanically movable relative to each other, in order to modify the angular orientation of the x-rays relative to the lamellae. In particular, the grating device can be adapted to tilt the lamellae for modifying the angular orientation of the x-rays 21 relative to the lamellae. Thus, as an alternative to or in addition to moving the focal spot, the lamellae, i.e. the grating comprising the lamellae, may be tilted, in order to modify the angular orientation of the x-rays relative to the lamellae. Moreover, the lamellae may be movable from a traversing position, in which the x-rays traverse the lamellae, to a non-traversing position, in which the x-rays do not traverse the lamellae, and vice versa. This is schematically and exemplarily illustrated in Fig. 8.

Fig. 8 schematically and exemplarily shows a grating 41 of the grating device 3, wherein the grating 41 comprises the lamellae with the spacers. The grating device 3 further comprises a moving unit 40 like a motor for moving the grating 41 out of the x-rays 21 in the direction indicated by the arrow 42. The moving unit 40 can also be used to tilt the grating 41 for modifying the angular orientation of the x-rays 21 relative to the lamellae of the grating. However, for this tilting also another moving unit, particularly another motor, may be used. Thus, the angulation of the grating relative to the beam formed by the x-rays can also be changed by mechanically moving the grating. It is also possible that in addition to or instead of moving the grating the x-ray source 2 or a component of the x-ray source 2 is mechanically moved for changing the angulation between the grating and the beam formed by the x-rays.

By moving the grating 41 into or out of the beam formed by the x-rays, projections can be acquired with and without the grating 41. The computed tomography system 1 can therefore be operated in different acquisition modes, wherein in one acquisition mode the grating 41 is used and in another acquisition mode the grating 41 is not used.

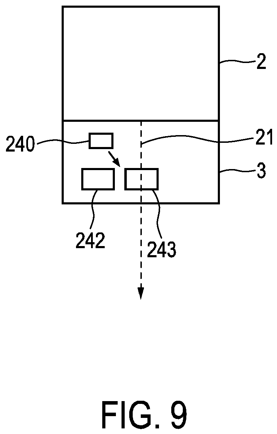

In a further embodiment, which is schematically and exemplarily illustrated in Fig. 9, the grating device 3 comprises two different gratings 242, 243, wherein the lamellae of the different gratings 242, 243 have different properties, for instance, different thicknesses, different heights, different periods, i.e. different distances between the lamellae, and/or different materials. Moreover, in this embodiment the grating device 3 comprises a moving unit 240 like a motor for moving the gratings 242, 243 such that one of these gratings 242, 243 or no grating is traversed by the x-rays 21 generated by the x-ray source 2. The different gratings 242, 243 have different properties such that they attenuate the x-rays 21 differently, which leads to different energy spectra. Thus, by changing which of the gratings 242, 243 is traversed by the x-rays 21 the energy spectrum of the x-rays traversing the person to be imaged can be modified. This can lead to an increased range of providable energy spectra of the x-rays.

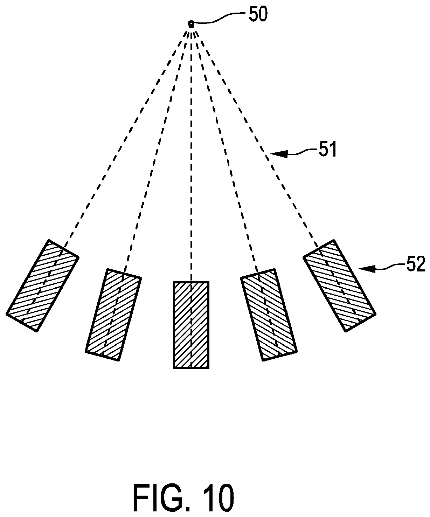

As schematically and exemplarily illustrated in Fig. 10, in an embodiment the several gratings comprise diverging lamellae 52 such that fictive extensions 51 of the lamellae 52 meet at a respective fictive lamellae focus point 50, wherein different gratings, i.e. different sets of lamellae, can have a same lamellae focus point or different lamellae focus points. Thus, the gratings, which can also be regarded as being absorber gratings, can have lamellae which are oriented such that projections of these lamellae meet in one point, i.e. at the lamellae focus point 50. If another grating has another lamellae focus point which is closer to the lamellae, in Fig. 10 the lamellae 52 would be more divergent. If the lamellae focus point would be farther away from the lamellae 52, the lamellae 52 would be more parallel. The grating device can comprise several different sets of lamellae having different degrees of divergence. It should be noted that Fig. 10 is only used for illustrative purposes and that preferentially the divergence of the lamellae is much smaller, i.e. the distance between the lamellae and the lamellae focus point in relation to the height of the lamellae is preferentially much larger. For instance, the height of the lamellae might be in the millimeter range, i.e., for instance, smaller than 5 mm, and the distance between the lamellae and the lamellae focus point might be in the centimeter or decimeter range, i.e., for instance, larger than 5 cm or larger than 10 cm. However, the dimensions might also be different.

The grating device 3 may comprise several gratings with lamellae focus points at different positions on the metal surface of the anode, wherein the x-ray source may be adapted to move the focal spot from a position, at which it meets the lamellae focus point of one of the sets of lamellae, to another position, at which it meets the lamellae focus point of another of the sets of lamellae, and vice versa, in order to select the grating to be used for filtering the x-rays without necessarily requiring mechanical movements. This allows for a very fast selection of the grating to be used for filtering the x-rays, wherein also in this example the gratings preferentially have different properties, in order to attenuate the x-rays differently, thereby further increasing the range of possible energy spectra. In an embodiment the x-ray source and the grating device are configured such that it is not selected whether only one of the gratings is traversed, but it is only selected which grating is predominantly traversed, wherein the grating, which is predominantly traversed, is the grating of which the lamellae focus point currently meets the focal spot. Also by changing which grating predominantly influences the absorption of the x-rays the energy spectrum of the x-rays can be modified, in order to increase the range of providable energy spectra.

In an embodiment the imaging system is adapted to generate an image of a contrast agent, wherein the projections are generated by using two different gratings, wherein the K-edge energy of a first grating is smaller than the K-edge energy of the contrast agent and wherein the K-edge energy of a second grating is larger than the K-edge energy of the contrast agent. Preferentially, the first and second gratings are tin and gold gratings and the contrast agent is iodine.

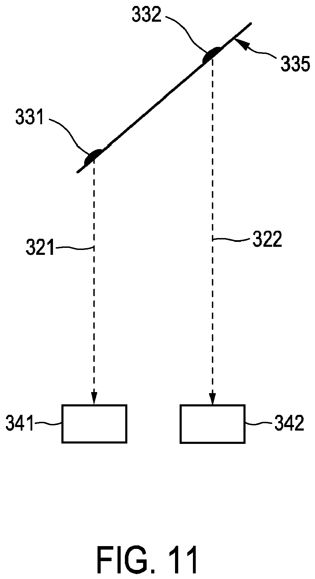

The selection of a grating 341, 342 by matching the position of the focal spot with a respective position of a respective lamellae focus point is schematically and exemplarily illustrated in Fig. 11. In Fig. 11 the focal spot can be moved from a position 331 on the surface 335 of the anode to the position 332 and vice versa, wherein these two positions 331, 332 coincide with the lamellae focus points of the gratings 341, 342, respectively. If the focal spot is at the position 331, x-rays 321 are generated which are only or mainly filtered by the grating 341. If the focal spot is at the position 332, x-rays 322 are generated which are only or mainly filtered by the grating 342. It should be noted that Fig. 11 is of course just an illustrative figure, wherein preferentially the x-rays are divergent and the gratings 341, 342 have divergent lamellae having lamellae focus points at the positions 331, 332, respectively.

The x-ray source can also be adapted to change the voltage and/or the current between the cathode and the anode for changing the energy spectrum of the x-rays, in order to further increase the range of providable energy spectra of the x-rays. This can be done dynamically, i.e. during the acquisition of detection values, in order to increase the spectrum of the spectral projections. The voltage, which can be changed, can be regarded as being an acceleration voltage and the current can be regarded as being the x-ray source current of the x-ray source.

The switched filter characteristics of the grating, i.e. the changes of the angular orientation, may result in significant changes of the x-ray flux, i.e. photons per period time, and therefore different signal-to-noise ratios (SNRs) of the acquired projections. This may negatively affect the quality of the output images. It is therefore preferred to have roughly the same number of photons and SNR for the spectrally coded measurements. Thus, it is preferred to modify the x-ray tube current and/or the acquisition times during which detection values are acquired for a respective energy distribution of the x-rays depending on the overall absorption of the grating in the respective angular orientation. This modification of the x-ray tube current and/or the acquisition times is preferentially carried out such that differences in the number of photons caused by different overall absorptions of the grating in the different angular orientations are reduced, particularly minimized. Thus, the SNR balance can be improved, for instance, by either increasing the x-ray tube output flux by increasing the x-ray tube current for measurements with strong absorption from the grating or by extended acquisition times to roughly collect the same number of photons for different angular orientations. If in addition to the switched filter characteristics other x-ray generation parameters of the x-ray source are dynamically changed like the tube voltage, the impact on the output flux should be added to the SNR balance considerations.

The spectral computed tomography system 1 further comprises a reconstruction unit 10 for reconstructing the image of the person based on the spectral projections. In this embodiment the reconstruction unit 10 is adapted to use a filtered back projection algorithm for reconstructing the image of the person. The reconstruction unit 10 can be adapted to use a material decomposition technique, in order to reconstruct different images, which correspond to different materials within the person, based on the spectral projections. For instance, if a contrast agent has been injected into the person, a first image can be reconstructed only showing the contrast agent within the person and a second image can be reconstructed showing the person without the contrast agent. The reconstruction unit 10 can also be adapted to reconstruct different images, which correspond to different physical effects like the Compton effect and the photoelectric effect, based on the spectral projections. The reconstruction unit 10 can of course also be adapted to use other spectral reconstruction techniques. Known reconstruction techniques, which might be used by the reconstruction unit, include K-edge imaging reconstruction techniques and are disclosed, for instance, in the article "K-edge imaging in x-ray computed tomography using multi-bin photon counting detectors" by E. Roessl and R. Proksa, Physics in Medicine and Biology, volume 52, pages 4679 to 4696 (2007) which is herewith incorporated by reference. Since the reconstruction unit 10 finally generates the image based on the spectral projections, it can be regarded as being an image generating unit.

The spectral computed tomography system 1 further comprises an input unit 12 like a keyboard, a computer mouse, a touchpad, et cetera and a display 13 for displaying the reconstructed image.

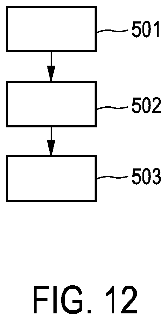

In the following an embodiment of an imaging method for generating an image of an object will exemplarily be described with reference to a flowchart shown in Fig. 12.

The imaging method for generating an image of an object is, in this embodiment, a spectral computed tomography imaging method for generating an image of a person. In step 501 the x-ray device 20 rotates around the rotational axis R and the person is not moved, i.e. the x-ray device 20 travels along a circular trajectory around the person. In another embodiment the x-ray device 20 can move along another trajectory, for example, along a helical trajectory, relative to the person. The x-ray device 20 provides x-rays traversing the person and the x-rays, which have traversed the person, are detected by the detector 6, in order to generate projections. While rotating the x-ray device 20 around the person, the angular orientation of the x-rays relative to the lamellae of the grating device of the x-ray device is modified, in order to generate projections which correspond to different energy distributions. Thus, the angular orientation is dynamically modified, in order to generate spectral projections by changing the energy spectrum of the x-rays provided by the x-ray device 20. This part of step 501 can be regarded as defining a method for providing x-rays by using the x-ray device 20. In step 502 an image of the person is reconstructed based on the spectral projections, wherein in step 503 the reconstructed image is shown on the display 13.

Spectral x-ray imaging adds clinically important information to conventional imaging, wherein this information is acquired by measuring tissue absorption with different x-ray spectra. These different x-ray spectra are generated by dynamically changing the direction of the x-rays generated by the x-ray source relative to the lamellae of the grating.

The favored geometry of the grating can be derived from several imaging system aspects and from restrictions of the grating manufacturing process. The main parameters are the thickness and height of the absorbing lamellae and the pitch. Some exemplary design consideration include a) grating manufacturing enables a limited set of absorber materials and a minimal thickness, b) the aspect ratio, i.e. grating height over pitch, in combination with the distance of the grating to the focal spot defines the required spatial movement of the focal spot, wherein restrictions of the x-ray source and the basic geometry of the imaging system will set constrains, c) the pitch of the grating should be small enough to ensure that the projection of the discrete grating lamellae onto the detector does not produce interferences of the gratings and the detector pixels, i.e. the grating structure should be "smeared out" on the detector, and d) the lamella thickness in combination with the absorber material defines the spectral characteristics and the total absorption of the grating. A typical embodiment for a medical computed tomography system may have tin lamellae with a thickness of, for instance, 10 $\mu$m and a height of, for instance, 2 mm. The grating period might be, for example, 50 $\mu$m.

Although in the embodiment described above with reference to Fig. 1 the x-ray device is used in a spectral computed tomography system, in other embodiments the x-ray device can be used in other x-ray systems needing different energy distributions, i.e. different energy spectra, of x-rays. In particular, the concept of modifying the angular orientation of the x-rays relative to the lamellae of the grating for changing the energy spectrum can be applied in other medical or non-medical, particularly industrial, x-ray imaging systems. For instance, an x-ray device providing different energy spectra of x-rays by modifying the angular orientation of the x-rays relative to lamellae of a grating can also be used in a single projection imaging system like a radiography imaging system, a dynamic projection imaging system like a fluoroscopy imaging system, a limited angle tomography imaging system like a tomosynthesis imaging system, et cetera. Moreover, as described above with reference to Fig. 1, the x-ray device can also be used in a tomographic imaging system, wherein the tomographic imaging system might be a two-dimensional computed tomography system or a three-dimensional computed tomography system.

In an embodiment the spacers can comprise or be made of a non-metal material or a combination of non-metal materials having a density being smaller than 3 g/cm$^3$. In an embodiment the spacers comprise or are made of Delrin or Teflon, which are characterized by a relatively low density and a relatively low effective atomic number.

Although in the embodiments described above with reference to Figs. 1 to 11 the spacers between the lamellae have only a very small x-ray absorption, the spacers can also have a significant x-ray absorption which, however, is still much smaller than the absorption of the absorber lamellae. In an embodiment the lamellae are made of or comprise a material having a K-edge energy within a range from 30 keV to 65 keV. Moreover, preferentially the lamellae are made of or comprise metal. In particular, preferentially the lamellae are made of or comprise metal having a K-edge energy within a range from 30 keV to 65 keV. The lamellae can be made of or comprise, for instance, at least one element of the group consisting of tin, gadolinium, molybdenum, gold, lead. For example, a grating can comprise tin lamellae and Delrin or Teflon spacers. The absorber lamellae can of course also comprise or be made of another x-ray absorbing material, particularly another metal material. Also the spacers can be made or comprise another material, particularly another non-metal material.

The x-ray device can be operated to provide a sequence of different output spectra of the x-rays, wherein for providing the different output spectra the angular orientation between the x-rays and the lamellae of the grating can be modified. In addition, further parameters can be modified for modifying the energy spectrum. For instance, x-rays can be generated with the grating within an x-ray beam and without the grating within the x-ray beam. Moreover, the used grating can be exchanged. For instance, for generating a certain energy spectrum a tin grating can be used, wherein the lamellae are tin lamellae, and for generating another energy spectrum a gold grating can be used, which has gold lamellae. Furthermore, in an embodiment, for generating a certain energy spectrum a grating with absorbing lamellae can be used together with a relatively high acceleration voltage of the x-ray source and for generating another energy spectrum the grating might not be used and the acceleration voltage might be lowered.

The x-ray device can be adapted to not provide x-rays, while the energy spectrum is modified, i.e., for instance, while there is a transition in a sequence of different output spectra. For instance, the x-ray generation can be disabled during these transitions, in order to avoid unwanted output spectra during these transitions or to avoid excessive anode track power loads. For disabling the x-ray generation known techniques can be used like tube grid switching.

If the x-ray device provides a sequence of output spectra, these output spectra are preferentially temporally alternately provided. The time periods, during which the different output spectra are provided, can be different. In particular, these time periods can be chosen to balance the integrated flux. Different x-rays, which have different energy spectra, can also have different intensities, wherein the difference in the intensity of x-rays having different energy spectra can be reduced by adjusting the time periods, at which the respective energy spectrum is provided, accordingly. For instance, if the intensity of x-rays having a first energy spectrum is larger than the intensity of x-rays having second energy spectrum, the x-rays having the first energy spectrum can be provided for a smaller time period than the x-rays with the second energy spectrum.

The detector used for detecting the x-rays can be a non-spectral detector, wherein still spectral detection values can be generated, because the energy spectrum of the x-rays detected by the detector is modified. However, the detector can also be a spectral detector, i.e. an energy selective detector, such as a dual layer or photon counting detector. This can lead to an increase of the number of sets of detection values which correspond to different energies or different distributions.

The spectral detection values are indicative of the energy dependence of the absorption of the x-rays by the object. The detection values can therefore also regarded as being spectral absorption data, i.e. the x-ray device can be used for generating spectral absorption data.

In an embodiment the imaging system is a computed tomography system being adapted to provide K-edge imaging. In this case the x-ray device and the detector are adapted to provide detection values for at least three different energies or energy distributions, i.e. at least three differently coded spectral radiation x-ray detection data are preferentially provided, if the imaging system is a K-edge imaging system.

Although in above described embodiments the object to be imaged is a person, in other embodiments the object to be imaged can of course also be another object like an animal or non-living object, particularly if the x-ray device is used for industrial imaging. The x-ray device can also be used for security imaging, for instance, for inspecting baggage.

The gratings can be regarded as being filters for filtering the x-rays. The imaging system, particularly the x-ray device, can have one or more additional filters for filtering the x-rays before reaching the object to be imaged. For instance, a bowtie filter, a pre-hardening filter to remove very low energy photons, a heel filter to correct for the heel effect, i.e. for a non-constant intensity along the cone direction, if the x-rays form a cone beam, et cetera.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Operations like the reconstruction of the computed tomography image performed by one or several unit or devices can be performed by any other number of units or devices. These operations and/or the control of the x-ray device in accordance with the method for providing x-rays and/or the control of the imaging system in accordance with the imaging method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

**Claims**

1. An x-ray device for providing x-rays, the x-ray device (20) comprising:

   - an x-ray source (2) for generating x-rays (21), and
   - a grating device (3) for absorbing at least a part of the x-rays (21), wherein the grating device (3) has lamellae (22) comprising an x-ray absorbing material,

   wherein at least one of the x-ray source (2) and the grating device (3) is adapted for modifying an angular orientation of the x-rays (21) relative to the lamellae (22).

2. The x-ray device of claim 1, wherein the at least one of the x-ray source (2) and the grating device (3) is adapted to modify the angular orientation such that a variation of penetration lengths through the lamellae (22) of all generated x-rays is minimized.

3. The x-ray device of claim 1, wherein at least one of the x-ray source and the grating device is adapted for setting an angular orientation of the x-rays relative to the lamellae, which is selected from a group of predefined angular orientations, wherein for each angular orientation of this group the variation of penetration lengths through the lamellae of all generated x-rays has a local minimum.

4. The x-ray device of claim 1, wherein at least one of the x-ray source and the grating device is adapted for setting an angular orientation of the x-rays relative to the lamellae, which is selected from a group of predefined angular orientations, wherein the angular orientation of a respective group is defined by

$$\alpha = \tan(np/h) \qquad,$$

wherein $\alpha$ is the angle enclosed by a) an x-ray of the generated x-rays and b) the lamellae, $n$ is a natural number, $p$ is the pitch of the grating and $h$ is the height of the lamellae.

5. The x-ray device of claim 1, wherein the x-ray source (2) comprises a focal spot (31) on a metal surface (35), from which the x-rays (21) emanate, wherein the x-ray source (2) is adapted to move the focal spot (31) for modifying the angular orientation of the x-rays (21) relative to the lamellae (22).

6. The x-ray device of claim 5, wherein the x-ray source (2) comprises an electron beam generation unit (30, 32) for generating an electron beam (33) directed on a region on the metal surface (35), thereby generating the focal spot (31) from which the x-rays (21) emanate, wherein the x-ray source (2) is adapted to move the focal spot (31) by moving the electron beam (33).

7. The x-ray device of claim 5, wherein the x-ray source (2) comprises an electron beam generation unit (30, 132, 142) for generating several electron beams (133, 143) and a switch (136, 146) for switching between the several electron beams (133, 143), wherein the different electron beams (133, 143) are directed on different regions on a metal surface (35) for generating different focal spots (131, 141), from which the x-rays (21) are emanatable, at different locations, wherein the x-ray source (2) is adapted to provide a movable focal spot by switching between the different electron beams (133, 143).

8. The x-ray device of claim 1, wherein the x-ray source (2) and the lamellae (22) are mechanically moveable relative to each other, in order to modify the angular orientation of the x-rays (21) relative to the lamellae (22).

9. The x-ray device of claim 1, wherein the x-ray source (2) comprises a focal spot (31) on a metal surface (35), from which the x-rays (21) emanate, wherein the grating device comprises several sets of lamellae, wherein the lamellae of each set diverge such that fictive extensions of the lamellae meet at a respective fictive lamellae focus point, wherein the lamellae (22) focus points are on the metal surface (35) and wherein the x-ray source (2) is adapted to move the focal spot (31) from a position, at which it meets the lamellae focus point of one of the sets of lamellae, to another position, at which it meets the lamellae focus point of another of the sets of lamellae, and vice versa.

**10.** The x-ray device of claim 1, wherein the grating device comprises several sets of lamellae, wherein the lamellae of different sets have different properties.

**11.** An imaging system for generating an image of an object, the imaging system comprising:

- an x-ray device (20) as defined in claim 1 for providing x-rays for traversing the object,
- an x-ray detector (6) for generating detection data depending on the x-rays after having traversed the object, and
- an image generating unit (10) for generating the image based on the detected x-rays.

**12.** A method for providing x-rays by using the x-ray device as defined in claim 1, the method comprising generating x-rays (21) by the x-ray source (2), wherein an angular orientation of the x-rays (21) relative to the lamellae (22) is modified.

**13.** An imaging method for generating an image of an object, the imaging method comprising:

- providing x-rays for traversing the object by an x-ray device (20) as defined in claim 1,
- generating detection data depending on the x-rays after having traversed the object by an x-ray detector (6), and
- generating the image based on the detected x-rays by an image generating unit (10).

**14.** A computer program for controlling an x-ray device as defined in claim 1, the computer program comprising program code means for causing the x-ray device (20) to carry out the method as defined in claim 12, when the computer program is run on a controller controlling the x-ray device (20).

**15.** A computer program for controlling an imaging system as defined in claim 1, the computer program comprising program code means for causing the imaging system (1) to carry out the imaging method as defined in claim 13, when the computer program is run on a controller controlling the imaging system (1).

FIG. 1

21

24

23

22

25

26

FIG. 2

FIG. 3

FIG. 4

**FIG. 5**

**FIG. 6**

FIG. 7

FIG. 8

FIG. 9

FIG. 10

FIG. 11

FIG. 12

Europäisches
Patentamt

European
Patent Office

Office européen
des brevets

# EUROPEAN SEARCH REPORT

Application Number

EP 17 16 1362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/032864 A1 (KONINKLIJKE PHILIPS NV [NL]) 2 March 2017 (2017-03-02) * page 9, line 29 - page 16, line 8; figures 1,2 * ----- | 1-3, 11-15 | INV. A61B6/03 A61B6/00 G21K1/10 |
| X | US 2010/008472 A1 (BOHN ROBERT [DE] ET AL) 14 January 2010 (2010-01-14) * paragraph [0003]; figures 1-6 * * paragraph [0010] - paragraph [0011] * ----- | 1,11-15 | |
| X | WO 2016/020178 A1 (KONINKL PHILIPS NV [NL]) 11 February 2016 (2016-02-11) * page 4, line 29 - page 5, line 30; figures 2-4 * ----- | 1,11-15 | |
| X | US 2012/057677 A1 (VOGTMEIER GEREON [DE] ET AL) 8 March 2012 (2012-03-08) * paragraph [0031] * * paragraph [0096] - paragraph [0097] * ----- | 1,11-15 | |
| | | | TECHNICAL FIELDS SEARCHED (IPC) A61B G21K |

~~The present search report has been drawn up for all claims~~

| Place of search | Date of completion of the search | Examiner |
|---|---|---|
| Munich | 20 September 2017 | Martinez Möller, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4, 11-15

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 17 16 1362

The Search Division considers that the present European patent application does not comply with the requirements of unity of invention and relates to several inventions or groups of inventions, namely:

1. claims: 1-4, 11-15

   Details of modifying the angular orientation of the x-rays relative to the lamellae to particular values, thus allowing to improve minimise the variation of penetration lengths through the lamellae.
   ---

2. claims: 5-9

   Details of the techniques used to modify the angular orientation, thus allowing to avoid mechanical movements (by moving focal sport) and/or achieving large change angles (by mechanical movement).
   ---

3. claim: 10

   Details of using different sets of lamellae, thus allowing to provide a larger range of different x-ray spectra.
   ---

## ANNEX TO THE EUROPEAN SEARCH REPORT
## ON EUROPEAN PATENT APPLICATION NO.

EP 17 16 1362

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2017

| Patent document cited in search report | | Publication date | Patent family member(s) | | Publication date |
|---|---|---|---|---|---|
| WO 2017032864 | A1 | 02-03-2017 | CN | 107072621 A | 18-08-2017 |
| | | | JP | 2017530827 A | 19-10-2017 |
| | | | WO | 2017032864 A1 | 02-03-2017 |
| US 2010008472 | A1 | 14-01-2010 | DE | 102006039793 B3 | 24-01-2008 |
| | | | US | 2010008472 A1 | 14-01-2010 |
| | | | WO | 2008022879 A1 | 28-02-2008 |
| WO 2016020178 | A1 | 11-02-2016 | CN | 106575533 A | 19-04-2017 |
| | | | EP | 3178089 A1 | 14-06-2017 |
| | | | JP | 6247423 B2 | 13-12-2017 |
| | | | JP | 2017522125 A | 10-08-2017 |
| | | | US | 2017202528 A1 | 20-07-2017 |
| | | | WO | 2016020178 A1 | 11-02-2016 |
| US 2012057677 | A1 | 08-03-2012 | CN | 102460237 A | 16-05-2012 |
| | | | EP | 2443491 A1 | 25-04-2012 |
| | | | JP | 2012530270 A | 29-11-2012 |
| | | | JP | 2017012814 A | 19-01-2017 |
| | | | RU | 2012101313 A | 27-07-2013 |
| | | | US | 2012057677 A1 | 08-03-2012 |
| | | | WO | 2010146498 A1 | 23-12-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **E. ROESSL ; R. PROKSA.** K-edge imaging in x-ray computed tomography using multi-bin photon counting detectors. *Physics in Medicine and Biology,* 2007, vol. 52, 4679-4696